Europäisches Patentamt

(19) European Patent Office

Office européen des brevets

(11) Publication number: **0 037 723**
A2

(12) **EUROPEAN PATENT APPLICATION**

(21) Application number: **81301460.2**

(22) Date of filing: **03.04.81**

(51) Int. Cl.³: **C 12 N 15/00**
**C 07 H 21/04, C 12 P 21/02**
**C 12 N 1/20, C 12 N 5/00**

---

(30) Priority: **07.04.80 US 137758**

(43) Date of publication of application:
**14.10.81 Bulletin 81/41**

(84) Designated Contracting States:
**AT BE CH DE FR GB IT LI LU NL SE**

(71) Applicant: **THE REGENTS OF THE UNIVERSITY OF CALIFORNIA**
**2200 University Avenue**
**Berkeley, California 94720(US)**

(72) Inventor: **Bell, Graeme**
**1611 Funston Avenue**
**San Francisco California 94122(US)**

(72) Inventor: **Goodman, Howard Michael**
**3006 Clay Street**
**San Francisco California 94155(US)**

(72) Inventor: **Rutter, William J.**
**80 Everson Street**
**San Francisco California 94131(US)**

(72) Inventor: **Cordell, Barbara**
**25 Priest Street**
**San Francisco California(US)**

(72) Inventor: **Laub, Orgad**
**1381 7th. Avenue**
**San Francisco California 94122(US)**

(72) Inventor: **Rall, Leslie**
**22 Dellbrook**
**San Francisco California(US)**

(72) Inventor: **Pictet, Raymond L. Institute de Recherche en Biologie Moleculaire University Paris 7 Tour 43**
**2, Place Jussieu F-74221 Paris Cedex 05(FR)**

(74) Representative: **De Minvielle-Devaux, Ian Benedict Peter et al,**
**CARPMAELS & RANSFORD 43, Bloomsbury Square London WC1A 2RA(GB)**

(54) **Expression of hormone genomic clones.**

(57) Cloned DNA segments comprise a gene coding for a human polypeptide hormone and are derived from human genome and are capable of being expressed in a eucaryotic cell. The human polypeptide hormone may be human insulin. The cloned DNA segments may be comprised in a DNA transfer vector.

./...

EP 0 037 723 A2

FIG. I

# EXPRESSION OF HORMONE GENOMIC CLONES

Genomic clones are purified DNA segments replicated directly from DNA of a source organism. Genomic clones are distinguishable from cDNA clones in that the latter are transcribed from isolated messenger RNA (mRNA). The distinction is not only opertionally but chemically significant, at least where the source organism is eucaryotic.

Recent advances have led to the realization that eucaryotes differ radically from procaryotes with respect to the structure and organization of eucaryotic DNA. In most eucaryotes, only a minor proportion of total DNA actually encodes specific amino acid sequences. Large untranslated regions have been known for some time to exist between coding segments. More recently, it has been discovered that coding segments themselves are interrupted by untranslated intervening sequences of substantial length. The biological and evolutionary significance of these intervening sequences is still a subject for peculation. However, a general understanding of the functional mechanics of transcription and translation of eucaryotes is beginning to emerge.

Genes containing intervening sequences, sometimes termed introns, are initially transcribed in their entirety in the nucleus of the eucaryotic cell. The initial mRNA transcripts are then edited or processed by unknown means to remove the intervening sequences and produce a coherent, uninterrupted mRNA coding sequence, which is transported to the cell cytoplasm. Additional mRNA processing steps occur, including a "capping" reaction that modifies the 5'-end and addition of a poly A sequence at the 3'-end. None of these processing steps is observed in procaryotes. For a general review of current understanding, see Dulbecco, R., Micriobiol. Revs. 43, 443 (1979).

A practical consequence, from the standpoint of recombinant DNA technology, is that genomic clones are not directly expressible by procaryotes, although conceivably they might be improperly expressed, introns and exons, to yield some sort of chimeric protein. Similarly, cloned cDNA, which is expressible in procaryotes, may not be efficiently expressed, if at all, in eucaryotic hosts.

Therefore, where expression by a eucaryotic host is desired, the use of genomic clones is deemed to be the preferred technique.

Expression by eucaryotic hosts is useful in circumstances where the desired protein is initially expressed as a precursor protein which itself requires additional processing. Such process steps include the specific cleavage of the initial translation product or a sequence of such steps, addition of glycosyl residues at specific sites, and incorporation into dimers, tetramers or multimeric associations with other proteins. Expression in eucaryotic hosts permits these steps to be accomplished as part of the eucaryotic expression process. In contrast, expression in procaryotes frequently requires additional difficult and time-consuming in vitro steps of low yield to accomplish the same result. Alternatively, special modifications of the coding sequence must be made prior to transformation of the procaryotic host. Insulin, for example, is initially expressed as pre-proinsulin, a protein product carrying the A and B peptides of insulin, an intervening C peptide, and an N-terminal signal peptide. The latter peptide permits vectorial transport of the protein into the endoplasmic reticulum of pancreatic B cells, and is removed during this process. The C peptide is believed to function to permit folding of the protein such that the A and B peptide chains are properly juxtaposed for formation of the correct disulfide bridges in the mature insulin molecule. Procaryotic expression of insulin cDNA requires additional in vitro steps to carry out the necessary processing of the expression product. One prior art strategy has been the complete chemical synthesis of sequences coding for the A and B peptides, separate procaryotic expression of these peptides and in vitro joining of the peptides by chemical means (Goeddel, D.V., et al, Proc. Nat. Acad. Sci. USA, 76, 106 (1979). A separate strategy is based upon procaryotic expression of human proinsulin cDNA with correct joining of the disulfide pairs, a consequence of proper folding induced by the presence of the C peptide, followed by in vitro excision of the C peptide - see our co-pending European Patent application 80303195.4 filed on 11 September 1980.

In both cases, the normal processing functions of the eucaryotic cell are absent in the procaryotic host and must be supplied by less efficient _in vitro_ methods. Expression in eucaryotic cells therefore presents advantages for production of human insulin.

The human body produces a large variety of peptides and proteins which serve a regulatory function. These include for example insulin, growth hormone, ACTH, somatostatin, beta-endorphin, nerve growth factor, epithelial growth factor and the somatomedins. Normally, the production of such proteins is carried out by highly specialized cells which produce these substances in small and precisely controlled amounts. It would be desirable to obtain sufficient quantities of these substances for therapeutic purposes, to supplement their missing function and for the treatment of deficiency states. However, the feasibility of producing such a regulatory protein with the aid of eucaryotic cells transformed by a specific gene has never been demonstrated in the prior art.

Normally, human insulin is produced by the highly differentiated B cells of the Islets of Langerhans in the pancreas. The production of insulin is highly regulated, since it has powerful hormonal effects. Both over-production and under-production are harmful to the organism. The same is true for other regulatory hormones of the body, including the compounds discussed _supra_. Since somatic cells of the organism are believed to carry out the same genetic information, it is reasonable to expect that special regulatory mechanisms exist to prevent production of such hormones by all but the highly specialized endocrine cells. Production of insulin or other hormones by the corresponding specific cells is not practical due to the fact that differentiated cells are difficult to culture, or tend to lose their special synthetic capabilities after one or two generations. However, various stable cultured cell lines are known which have been maintained in culture for years. If such cells could be induced to express either their endogenous insulin gene or an exogenous, cloned insulin gene, the problems presented by procaryotic expression would be overcome. Even if a stable differentiated cell line could be found to produce the desired hormone, as occasionally occurs with certain tumours, the added flexibility to use modified DNA to produce a modified protein provides a unique advantage of the recombinant DNA approach.

The process of controlling the gene expression in eucaryotic cells is complex and imperfectly understood (see Dulbecco, R., _supra_). Virtually nothing at all is known about the special processes regulating hormone synthesis. The art of transforming eucaryotic cells is in its infancy. That transformation can occur is known. Very little is known about the fate of the transforming DNA, whether it becomes integrated in the host chromosome or replicates by the use of a transfer vector bearing its own replication origin.

Successful attempts have been made to transform mouse "L" cells, a well-etablished cell line derived from a mouse fibroblast tumour, Kit, S., et al, _Exp. Cell Res._, 31, 297 (1963). In that system, the cell strain lacks thymidine kinase activity (tk$^-$), but could be transformed to tk$^+$ by _Herpes simplex_ virus DNA coding for thymidine kinase. (Wigler, M., et al, _Cell_, 11, 223 (1977) ). Further studies showed that co-transformation with thymidine kinase DNA and DNA from bacteriophage ØX 174, plasmid pBR 322 or the cloned chromosomal rabbit beta globin gene indicate that cells which have received and expressed the thymidine kinase gene are at least capable of carrying the above-mentioned exogenous DNAs, Wigler, M., et al., _Cell_, 16, 777 (1979). Similarly, mutant "L" cells lacking both the thymidine kinase and adenine phosphoribosyltransferase (Ltk$^-$aprt$^-$) were transformed to express adenine phosphoribosyltransferase activity by unfractionated high molecular weight genomic DNA from Chinese hamster, human and mouse cells and by restriction endonuclease digested DNA from rabbit liver. However, in the absence of selection pressure, i.e. during growth in a medium

where functional adenine phosphoribosyltransferase was not required, some of the transformed strains lost the ability to express the transforming gene. More recently, expression of the chicken ovalbumin gene in mouse "L" cells (Ltk⁻aprt⁻) was achieved using a plasmid (pBR322) containing both a genomic clone of the ovalbumin gene, and three copies of the Herpes simplex virus thymidine kinase gene. (Lai, E.C., et al., Proc. Nat. Acad. Sci. USA, 77, 244 (1980) ). In that case, all of the transformants having thymidine kinase activity also synthesized chicken ovalbumin, as identified by immunoreactivity. However, the rate of ovalbumin synthesis varied over two orders of magnitude in independent isolates of transformed cells.

The widespread use of thymidine kinase stems from the fact that it is one of the few selection systems available for use with cultured cells. Cells lacking thymidine kinase are able to grow satisfactorily in a normal growth medium such as Dulbecco's modified Eagle's medium. However, functional thymidine kinase is required if the cells are to grow in medium supplemented by hypoxanthine, aminopterin and thymidine, commonly termed HAT. Following transformation, cells able to grow in HAT medium are those which have acquired the thymidine kinase gene and are expressing functional thymidine kinase. When it is desired to test for transformation by another gene for which no selection technique is known, the cells are co-transformed with a thymidine kinase gene to enable the investigator to identify transformed cells, by HAT selection. Generally, more extensive experimentation is required to demonstrate transformation by a gene not otherwise selected for.

Expression has also been attempted using a defective simian virus 40 (SV40 vector) containing a portion of the SV40 genome including the late promoter region. Mouse beta-globin DNA was joined with the SV40 vector and introduced into monkey kidney cells. The mouse gene was expressed at high frequency into mouse beta-globin. However, transcription appeared to have inititated at the SV40 late romoter region rather than the beta-globin gene promoter.

If initiated from the SV40 promoter, the expression product may have been a fusion protein or may have been otherwise inaccurately inititated. (Hamer, D.H., et al, Nature, 281, 35 (1979 ).

From the foregoing discussion of the prior art, it is concluded that, while expression of genomic DNA in eucaryotic cells is at least feasible incertain cases, little is known about the factors affecting the fate of the transforming DNA or the nature and amount of expression product formed. Specific results in the art remain highly unpredictable.

The present invention demonstrates for the first time the expression of a mammalian hormone in a eucaryotic cell into which has been transferred a genomic clone containing the gene for the hormone. In particular, mouse "L" cells have been transformed with the genomic clone of human insulin and have been shown to express a product immunologically reactive as insulin. Cells not so transformed fail to express the insulin product. In contrast to previously reported results, only a minor fraction of the cells expressing thymidine kinase were also discovered to express insulin. Since little is known currently of the biological rules governing the expression of hormone genes in cells not specialized for the production of that hormone, the isolation of clones expressing a hormone gene could not have been predicted. There was some variation in the amount of insulin expressed in the three insulin-expressing strains. However, it was possible to observe expression at the level of 1,000 to 2,000 insulin molecules per cell.

The present invention provides a genomic clone of a hormone, a plasmid transfer vector comprising said genomic clone, a microorgranism containing and replicating said transfer vector, a transformed mammalian cell line capable of expressing the hormone and a method of making a hormone utilizing such a transformed cell line.

The present invention demonstrates for the first time the feasibility of synthesizing a polypeptide hormone in eucaryotic cells grown in culture and transformed by a genomic clone comprising the gene for said hormone. The practice of the invention is illustrated by the cloning of human insulin DNA, its characterization showing extensive intervening sequences, and its expression in mouse Ltk⁻aprt⁻ grown in culture.

The genomic clone of the present invention and cells transformed thereby, capable of expressing the polypeptide or protein hormone and coded by said gene, are useful for synthesis of a polypeptide or protein hormone without resort to $\underline{in}$ $\underline{vitro}$ processing reactions. The invention also provides means for making cell lines suitable for implantation to supplement endogenous production of the hormone for therapeutic purposes. For example, many diabetics and pre-diabetics have functional B cells capable of supplying part of the patient's insulin needs and responsive to normal metabolic control mechanisms. Implanted cells modified according to the present invention could provide a basal level of insulin for such patients, saving their endogenous B cells for use during times of peak insulin demand. Such treatment is highly advantageous since it provides the patient with adequate levels of insulin responsive to normal physiological controls.

The cells used in the present experiments, Ltk⁻aprt⁻ are derived from Ltk⁻ clone D described by Kit, S., et al., $\underline{supra}$. The cells are maintained in Dulbecco's modified Eagle's medium containing 10% foetal calf serum, commercially available from Flow Laboratories, Rockville, Maryland. The Ltk⁻aprt⁻ cell strain is further described by Wigler, M. et al, $\underline{Proc. Nat. Acad. Sci.}$, 76, 1373 (1979).

Restriction endonucleases were obtained commercially from New England BioLabs, Cambridge, Massachusetts, and were used under reaction conditions specified in the manufacturer's instructions.

The invention is next illlustrated by the specific examples of cloning and expression of the human insulin gene. However, the invention is not intended to be limited to these specific examples and must be deemed broadly applicable to genomic expression of highly regulated proteins generally, especially polypeptide hormones.

The invention provides a cloned DNA segment derived from the human genome comprising the gene for human insulin and being capable of being expressed in a eucaryotic cell, a transfer vector comprising said cloned DNA, a micro-organism containing and replicating said transfer vector and a eucaryotic cell strain, transformed by the cloned gene and being capable of expressing said gene. The transfer vector, microorganism and eucaryotic cell strain have been placed on deposit with the American Type Culture Collection, 12301 Parklawn Drive, Rockville, Maryland. The deposited transfer vector designated pBR322/gHI12.5 as described in Example 1, and has ATCC accession number 40023. The microorganism E. coli HB101pBR322/gHI12.5, described in Example 1, has ATCC accession number 31605. The eucaryotic cell line, designated Ltk⁻aprt⁻gHI12.5 clone 6-4, described in Example 3, was given ATCC accession number CRL-8028.

EXAMPLE 1 - Expression of human genomic clones

A library of fragments of human foetal liver DNA has been cloned in bacteriophage lambda Charon 4A by Lawn, R.M., et al. Cell 15. 1157 (1978). A preparation of this phage was provided by Dr. T. Maniatis. The phages were plated on suitable indicator bacteria as described by Cordell, B., et al., Cell 18, 533 (1979) at a dilution yielding 104 plaques per plate. The plaques were screened for the presence of human insulin gene sequences by in situ hybridization (with radioactively labelled cloned cDNA coding for human preproinsulin - see our co-pending European Patent Application 80303195.4 filed on 11 September 1980). Approximately 10⁶ plaques were screened in this manner, of which two were found to contain DNA which hybridized with the probe. These clones were purified by growth of phage stocks derived from the relevant plaques and their DNA was further characterized by restricion enzyme analysis. The DNA of each of the phage stocks was digested with EcoRI, the digested DNA fractionated by gel electrophoresis and the fragments which contained insulin gene sequences were identified by hydridization with radiolabelled human insulin cDNA, using the method of Southern, E.M., J. Mol. Biol., 98, 503 (1975). The clones were found to contain the insulin gene fragments on EcoRI fragments of 12.5 kb, 14.1 kg and were designated gHI 12.5 and gHI 14.1, respectively. (Molecular size of DNA is conveniently measured by the length in units of 1,000 base pairs, abbreviated kb). Since the human DNA library was originally constructed from DNA fragments obtained by essentially random cleavage, the data suggested by the two clones represented overlapping regions of human DNA. This view was confirmed by further restriction mapping. For comparison purposes, a cloned cDNA fragment incorporating the entire human preproinsulin coding sequence is about 0.416 kb in length.

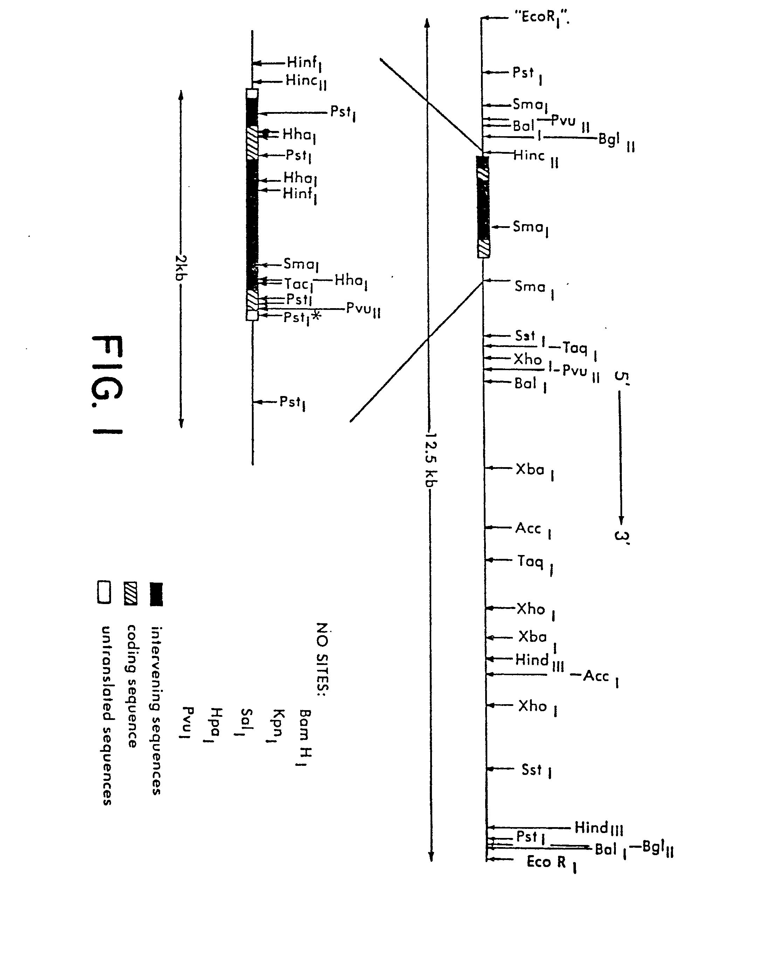

The 12.5 kb EcoRI fragment of gHI 12.5 was sub-cloned in plasmid pBR322 at the EcoRI site. The recombinant transfer vector was designated pBR322/gHI 12.5. The plasmid was replicated in E. coli HB101 transformed with bBR322/gHI 12.5, hence designated E. coli HB101 pBR322/gHI 12.5. Further restriction mapping, coupled with hybridization, made it possible to map the position of the insulin gene on the 12.5 kb fragment. The restriction mapping experiments indicated that the gene was not colinear with the mature mRNA, since the extent of the coding regions was greater than the 416 base pairs present in the cloned human insulin cDNA used as a probe. The exact location and structure of the gene was determined by DNA sequencing, as described below. Figure 1 shows a restriction map of the 12.5 kb fragment, showing the location of the insulin sequence and, in more detail, the general locations of coding sequences, intervening sequences and untranslated sequences in the insulin gene. It can be seen, for example, that the enzyme SmaI generated two fragments of about 900 and 1500 base pairs respectively, which hybridized with the cDNA probe. Since no SmaI sites were known in the cDNA sequence, it was concluded that the gene possesses an intervening sequence containing a SmaI site.

EXAMPLE 2

The human insulin gene was sequenced by the method of Maxam, A. and Gilbert, W., Proc. Nat. Acad. Sci. USA, 74, 560 (1977). The sequence determined extended from the PvuII site on the 5'-side of the gene, with respect to the mRNA, to a point 97 base pairs beyond the termination codon at the 3'-end. The sequence is shown in Table 1. In addition to the nucleotide sequence of the human gene and adjacent regions, the translation of the coding regions into preproinsulin is presented. The boxed sequence TATAAAG is commonly found near the 5'-starting point of transcription. The asterisk symbolizes the putative 5'-end of the mRNA, where the characteristic capping reaction of eucaryotic mRNA processing occurs. The arrow indicates the 3'-end of the mRNA, based upon homology with the rat insulin II gene, Lomedico, P., et al, Cell 18, 545 (1978). The identified intervening sequences are contained within parentheses. Every fiftieth base is numbered, the last digit of the number being placed over the corresponding base. The entire human preproinsulin sequence is contained within the coding region, which in turn is interrupted by a large intervening sequence within the region coding for the C-peptide.

EXAMPLE 3 - Expression of cloned human insulin gene

Expression of the human insulin genomic clone described in Example 1 was obtained by transformation of L cells in tissue culture. The fragment used for transformation was that designated gHI 12.5, carried on plasmid pBR322/gHI 12.5, replicated in E. coli HB101. The strategy of the experiment was simultaneously to transform the cells with a marker DNA of known transforming activity and with the insulin DNA. The insulin DNA was present in excess to increase the probability that cells transformed with marker would already have received the insulin gene as well.

Ltk⁻aprt⁻ cells used in these studies were obtained from Dr. Richard Axel of Columbia University. The cells were genetically deficient in thymidine kinase activity and in adenine phosphoribosyltransferase activity. The cells were cultured in DME medium supplemented with 10% (v/v) foetal calf serum, Dulbecco, R., Virology, 8, 369 (1959). The marker DNA codes for thymidine kinase and was derived from Herpes simplex virus, and was cloned in a 3.4 kb BamHI fragment. 64 ng of thymidine kinase plasmid, together with 26 µg of insulin plasmid were used to transform $10^6$ cells. Successful transformation was detected by the ability of cells to grow in the same growth medium, supplemented with $5 \times 10^{-4}$ M hypoxanthine, $4 \times 10^{-7}$ M aminopterin, and $1.6 \times 10^{-5}$ M thymidine, hereinafter termed HAT medium. The genetic defect of the L cells used herein is such that their survival in HAT medium depends upon an active thymidine kinase enzyme. Therefore, cells transformed with the Herpes viral thymidine kinase gene are able to form colonies. The transformation rate in the present experiment was about 40 colonies per $10^6$ cells.

The transformation was carried out essentially as described by Wigler, M., et al, Proc. Nat. Acad. Sci. USA, 76, 1373 (1979). Cells were seeded at a density of $2.5 \times 10^5$ per 100 mm dish for 48 hr. grown in fresh medium for 4 hr, and then inoculated with the described DNA mixture. After 48 hours' incubation at 37°C, the cells were then cultured in the HAT selection medium, which was changed every three days. After eighteen days, 20 HAT-resistant-colonies were selected. They were grown for several passages to accumulate material sufficient for DNA and insulin analysis.

Transformed cells were lysed in a proteinase K, sodium dodecyl buffer as described in Wigler, M., et al., Cell, 16, 777 (1979). The cell lysate was extracted first with an equal volume of buffer-saturated phenol and second with chloroform-isoamyl alcohol (24:1, v/v). High molecular weight DNA was isolated by mixing the aqueous phase with two volumes of cold ethanol and immediately removing the precipitate that formed. The DNA was dissolved in 1.0 mM Tris, 0.1 mM EDTA, pH 8.0. Agarose gel electrophoresis of restriction endonuclease-digested DNA and hybridization with labelled human insulin cDNA were performed as described by Southern, above. Endogenous (mouse) insulin DNA is distinguishable from human since the former is cleaved internally by EcoRI endonuclease while the latter is not. In addition, under stringent hybridization conditions, the human cDNA probe hybridizes preferentially with the human insulin DNA. Human insulin coding sequences were found in all of the transformed cell lines tested.

The expression of insulin by transformed cells was tested by a radioimmunoassay method described by Rall, L.B., et al., Endocrinology, 105, 835 (1979). The transformed cells were harvested, sonicated in acid-ethanol, and lyophilized, as described by deGasparo, M. et al,, Develop. Biol., 47, 106 (1975). The cell extracts were redissolved in distilled water and assayed as described.. Three of 20 thymidine kinase-positive clones were found to produce insulin, or an insulin-related molecule immunologically reactive with insulin antibody. Estimates of the amount of insulin made were based upon reactivity of known quantities of pork insulin in the assay. The most active cell line, designated Ltk⁻aprt⁻gHI 12.5 clone 6-4, produced about 1500 insulin molecules per cell. The fact that most transformed clones did not produce detectable insulin was in marked contrast to results reported by Lai, H.C. et al., Proc. Nat. Acad. Sci. USA, 77244 (1980) and suggest that factors necessary for obtaining expression of particular genes in eucaryotic cells are not well defined.

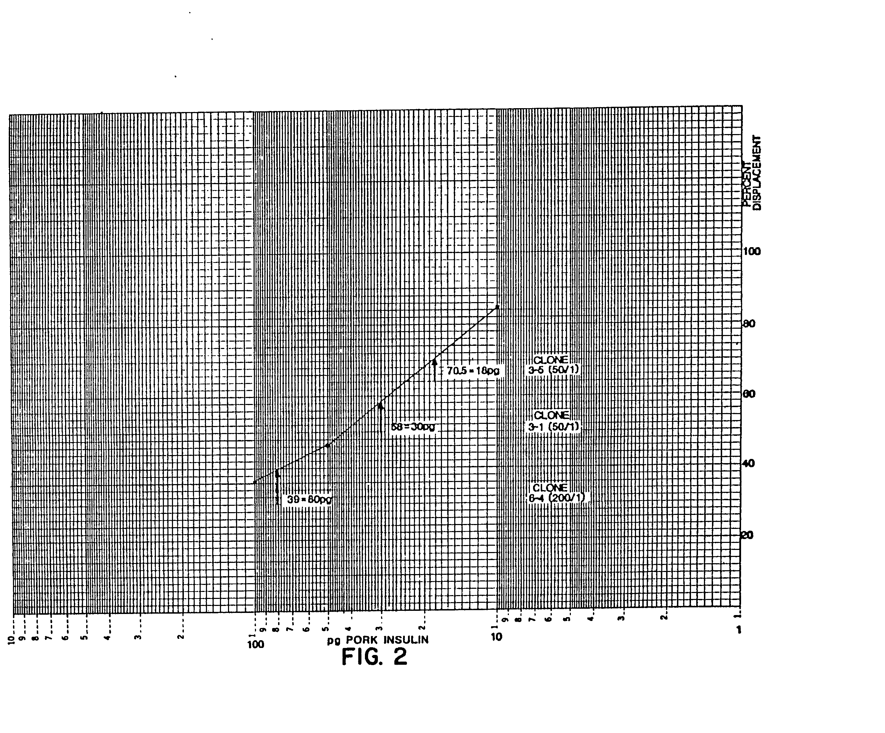

Figure 2 shows the radioimmunoassay results for the three insulin-producing clones. The results are plotted as the percent of·radioactive standard displaced as a function of the amount of insulin in the sample. The solid line is a standard curve based on porcine insulin. The arrows indicate results obtained with three different clones, at the indicated dilutions.. Assuming that the activity per molecule of the synthesized product is equivalent to that of pork insulin, the transformed cells were calculated to produce about 1,000 molecules per cell of the expression product. Control cells containing no human insulin sequences did not produce this product. Based upon its immunochemical reactivity and its expression in response to the presence of the human insulin gene, the expression product is considered to be identical with or closely related to human insulin.

EXAMPLE 4

The expression of a human insulin genomic clone was obtained using an SV40 cloning vector. For the purpose of this experiment, the 12.5 kb fragment of human DNA containing the insulin gene was cleaved with restriction enzymes XhoI in BglII. Seven DNA fragments were generated, and the 3 kb fragment containing the insulin gene was fractionated and purified by preparative gel electrophoresis. The 3 kb fragment was treated with Sl nuclease and DNA polymerase I to provide blunt ends, to which were attached HindIII oligonucleotide linkers. The fragment was then sub-cloned at the HindIII site of pBR322.

SV 40 DNA, 100 mg. was digested with HindIII and TaqI endonucleases to yield several fragments, one of which contains genetic determinants for the origin of SV 40 DNA replication and the promoter for late SV 40 genes, Laub et al., Virology 92, 310 (1979). This fragment, termed the SV 40 O/p fragment, was purified by preparative gel electrophoresis, and was similarly cloned at the HindIII site of pBR 322.

Purified sub-cloned insulin fragment, 3μg, was mixed with a two-fold molar excess of purified sub-cloned SV40 O/P fragment in a DNA ligase catalyzed joining reaction. The reaction products were fractionated according to size, and circular DNA composed of two repeats of the SV40 O/P fragment covalently linked to an insulin DNA segment was selected. This DNA was designated SV40/insulin DNA.

The replication of SV40/insulin recombinant is achieved by cotransfection of monkey cells with wild-type SV40 DNA, as described. Forty-eight hours post-cotransfection, viral DNA is extracted from infected cells by the Hirt procedure (Hirt, J. Mol. Biol., 26, 365 (1965) ). The procedure removes high molecular weight DNA, leaving SV40 wild-type and SV40/insulin DNA in the supernatant fraction. The DNA is fractionated by gel electrophoresis, both before and after treatment with endonuclease EcoRI. Wild-type SV40 DNA has an EcoRI cleavage site which is lacking in the SV40/insulin DNA. Similarly, the two can be distinguished on cesium chloride-

ethidium bromide gradients. A stock of SV40/insulin DNA is prepared from an EcoRI digest followed by fractionation and selection of closed circular DNA.

Monkey cells are transfected by DNA as described by Mertz and Berg, Virology, 62, 112 (1974). The cells are mixedly transfected with 3μg SV40/insulin DNA and 1 μg SV40 wild-type DNA. The cells are then examined 48 hours after transfection.

Transcription expression of the human insulin gene in cotransfected cells is detected by RNA blotting techniques. RNA is isolated separately from nuclear and cytoplasmic fractions of the co-infected cells. The RNA was denatured in glyoxal, fractionated by electrophoresis and analyzed for the presence of human insulin coding sequences by the method of Alwine, J.C., et al., Proc. Nat. Acad. Sci. USA, 74, 5350 (1977). Using labelled insulin cDNA as a probe, RNA corresponding to the insulin gene sequence is detected in co-transfected cells.

For the purpose of detecting the production of insulin, the radioimmunoassay described in Example 3 is employed. In addition, monkey cells transfected with the SV40/insulin DNA were labelled with 35S-methionine for 46 to 48 hours post-transfection.. Nuclear and cytoplasmic fractions are separated and the polypeptides are analyzed by sodium dodecylsulfate polyacrylamide gel electrophoresis. Individual protein bands are visualized by autoradiography. An additional band of protein the size of proinsulin is observed in the cytoplasmic fraction of cells transfected with the SV40/insulin DNA and in the incubation medium, but not in cells transfected with wild-type SV40, or non-transfected cells. These results are consistent with the conclusion that the SV40/insulin DNA serves as a transfer vector for the cloned human insulin gene and that expression of the gene in susceptible host cells can occur.

```
                                                              50
CAGCTGTGAGCAGGGACAGGTCTGGCCACCGGGCCCTTGGTTAAGACTCTAATGACCCGCTGGTCCTGAGG

                              100
AAGAGGTGCCGACGACCAAGGAGATCTTCCCACAGACCCAGCACCAGGGAAATGGGTCCGGAAATTGCAGC

        150                                              200
CTCAGCCCCCAGCCATCTGCCGACCCCCCCACCCCGCCCTAATGGGCCAGGCGGCAGGGGTTGACAGGTAG

                                    250
GGGAGATGGGCTCTGAGAC [TATAAAG] CCAGCGGGGGGCCCAGCAGCCCTC AGCCCTCCAGGACAGGCTG

        300                                                      350
CATCAGAAGAGGCCATCAAGCAG (GTCTGTTCCAAGGGCCTTTGCGTCAGGTGGGCTCAGGGTTCCAGGG

                                            400
TGGCTGGACCCCAGGCCCCAGCTCTGCAGCAGGGAGGACGTGGCTGGGCTCGTGAAGCATGTGGGGGTGAG

                        450
CCCAGGGGCCCCAAGGCAGGGCACCTGGCCTTCAGCCTGCCTCAGCCCTGCCTGTCTCCCAG) ATCACTG

        500   Met Ala Leu Trp Met Arg Leu Leu Pro Leu Leu Ala Leu Leu Ala
TCCTTCTGCC    ATG GCC CTG TGG ATG CGC CTC CTG CCC CTG CTG GCG CTG CTG GCC

Leu Trp Gly Pro Asp Pro Ala Ala Ala Phe Val Asn Gln His Leu Cys Gly Ser
CTC TGG GGA CCT GAC CCA GCC GCA GCC TTT GTG AAC CAA CAC CTG TGC GGC TCA

His Leu Val Glu Ala Leu Tyr Leu Val Cys Gly Glu Arg Gly Phe Phe Tyr Thr
CAC CTG GTG GAA GCT CTC TAC CTA GTG TGC GGG GAA CGA GGC TTC TTC TAC ACA

Pro Lys Thr Arg Arg Glu Ala Glu Asp Leu Gln V           700
CCC AAG ACC CGC CGG GAG GCA GAG GAC CTG CAG G . (GTGAGCCAACCGCCCATTGCTGC

                                    750
CCCTGGCCGCCCCCAGCCACCCCCTGCTCCTGGCGCTCCCACCCAGCATGGGCAGAAGGGGGCAGGAGGCT

        800                                                      850
GCCACCCAGCAGGGGGTCAGGTGCACTTTTTTAAAAAGAAGTTCTCTTGGTCACGTCCTAAAAGTGACCAG

                                            900
CTCCCTGTGGCCCAGTCAGAATCTCAGCCTGAGGACGGTGTTGGCTTCGGCAGCCCCGAGATACATCAGAG

                    950
GGTGGGCACGCTCCTCCCTCCACTCGCCCCTCAAACAAATGCCCCGCAGCCCATTTCTCCACCCTCATTTG

   1000                                           1050
ATGACCGCAGATTCAAGTGTTTTGTTAAGTAAAGTCCTGGGTGACCAGGGGTCACAGGGTGCCCCACGCTG

                            1100
CCTGCCTCTGGGCGAACACCCCATCACGCCCGGAGGAGGGCGTGGCTGCCTGCCTGAGCGGGCCAGACCCC

        1150                                     1200
TGTCGCCAGCCTCACGGCAGCTCCATAGTCAGGAGATGGGGAAGATGCTGGGGACAGGCCCTGGGGAGAAG

                            1250
TACTGGGATCACCTGTTCAGGCTCCCACTGTGACGCTGCCCCGGGGCGGGGGAAGGAGGTGGGACATGTGG

                1300
GCGTTGGGGCCTGTAGGTCCACACCCAGTGTGGGTGACCCTCCCTCTAACCTGGGTCCAGCCCGGCTGGAG

                                    1400
ATGGGTGGGAGTGCGACCTAGGGCTGGCGGGCAGGCGGGCACTGTGTCTCCCTGACTGTGTCCTCCTGTGT

                1450                                al Gly Gln Val
CCCTCTGCCTCGCCGCTGTTCCGGAACCTGCTCTGCGCGGCACGTCCTGGCAG)  TG GGG CAG GTG

Glu Leu Gly Gly Gly Pro Gly Ala Gly Ser Leu Gln Pro Leu Ala Leu Glu Gly
GAG CTG GGC GGG GGC CCT GGT GCA GGC AGC CTG CAG CCC TTG GCC CTG GAG GGG

Ser Leu Gln Lys Arg Gly Ile Val Glu Gln Cys Cys Thr Ser Ile Cys Ser Leu
TCC CTG CAG AAG CGT GGC ATT GTG GAA CAA TGC TGT ACC AGC ATC TGC TCC CTC

Tyr Gln Leu Glu Asn Tyr Cys Asn AM                      1650
TAC CAG CTG GAG AAC TAC TGC AAC TAG ACGCAGCCTGCAGGCAGCCCCACACCCGCCGCCT

                                    | 1700
CCTGCACCGAGAGAGATGGAATAAAGCCCTTGAACCAGC CCTGCTGTGCCGTTCTGTGTCTGGGGGCCCT

        1750
GGGCAAGCCCCACTTCCCCGGCACTGTTGTGAGCCCCTCCCAGCTCTCTCCACGCTCTCTCGGTGCC
```

CLAIMS

1.      A cloned DNA segment derived from the human genome, comprising the gene for a polypeptide hormone, capable of being expressed in a eucaryotic cell.

2.      The cloned DNA segment of claim 1, wherein the polypeptide hormone is insulin.

3.      The cloned DNA segment of claim 1, designated gHI 12.5.

4.      The cloned DNA segment of claim 1, designated gHI 14.1.

5.      A DNA transfer vector comprising the cloned DNA segment of claim 1.

6.      The DNA transfer vector of claim 5, wherein the cloned DNA segment comprises the human insulin gene.

7.      The DNA transfer vector of claim 6, comprising pBR322 with gHI 12.5 inserted at the EcoRI site thereof.

8.      A microorganism containing and replicating the DNA transfer vector of claim 1.

9.      A microorganism according to claim 8, wherein the transfer vector comprises a cloned DNA segment comprising the human insulin gene.

10.      The microorganism according to claim 9, wherein the transfer vector comprises the plasmid pBR322 with gHI 12.5 inserted at the EcoRI site thereof.

11.      The microorganism of claim 10, comprising E. coli RRI.

12.      A eucaryotic cell strain, grown in culture, transformed by the cloned DNA segment of claim 1, said cell strain capable of expressing said gene.

13.      The cell strain of claim 12, wherein the cloned DNA segment comprises the gene for insulin.

14.      The cell strain of claim 12 or 13, comprising mouse Ltk⁻aprt⁻ cells.

15.      The cell strain of claim 14, wherein the cloned DNA segment is selected from the group comprising gHI 12.5 and gHI 14.5.

16.      A cell culture comprising the eucaryotic cell strain of claim 12 and a growth medium suitable for growth and replication of said cell strain.

17.      A cell culture according to claim 16, wherein said cell strain is transformed by a cloned DNA segment comprising the gene for insulin.

18.     The cell culture of claim 16 or 17, comprising mouse Ltk⁻aprt⁻ cells.

19.     A process for making human insulin comprising the steps of:

    (a)  preparing the cloned DNA segment of claim 2, 3 or 4 containing the human insulin gene;

    (b)  transforming a eucaryotic cell strain grown in culture with said cloned DNA segment;

    (c)  selecting transformed cells capable of expressing said human insulin gene;

    (d)  making an extract of said cells; and

    (e)  purifying insulin from said extract.

20.     The DNA transfer vector of claim 6, comprising a portion of the SV40 genome and a portion of gHI 12.5 containing the insulin gene.

λ

CLAIMS

1. A process for preparing a DNA transfer vector for replicating a DNA segment containing a gene coding for a human polypeptide hormone comprising isolating a DNA segment containing said gene and inserting said segment into a DNA transfer vector characterized in that said DNA segment is derived from the human genome and said gene is capable of being expressed in a eucaryotic cell.

2. The process of claim 1, wherein the polypeptide hormone is insulin.

3. The process of claim 1, wherein the DNA segment is designated gHI 12.5.

4. The process of claim 1, wherein the DNA segment is designated gHI 14.5.

5. The process of claim 3, wherein gHI 12.5 is inserted at the EcoRI site of pBR323.

6. The process of claim 3, wherein the DNA transfer vector comprises a portion of the SV40 genome.

7. A process for preparing a microorganism containing and replicating a DNA transfer vector comprising transforming a microorganism with said transfer vector characterized in that said transfer vector is prepared according to any of claims 1 to 6.

8. The process of claim 7 wherein said microorganism is E. coli RRI.

9. A process for preparing a eucaryotic cell strain, grown in culture, containing a DNA transfer vector and capable of expressing said transfer vector comprising transforming a microorganism with said transfer vector, characterized in that said transfer vector is prepared according to any of claims 1 to 6.

10. The process of claim 9 wherein said cell strain comprises mouse Ltk⁻aprt⁻ cells.

11. A process for preparing a cell culture comprising a cell strain and a growth medium suitable for growth and replication of said cell strain characterized in that said cell strain is prepared according to claim 9 or 10.

12. A process for preparing human insulin comprising:

21

(a) preparing a transfer vector containing a gene for human insulin prepared according to any of claims 2 to 6;

(b) transforming a eucaryotic cell strain grown in culture with said transfer vector;

(c) selecting transformed cells capable of expressing said insulin gene;

(d) making an extract of said cells, and

(e) purifying human insulin from said extract.

# FIG. I

2kb

12.5 kb

Hinf I
Hinc II
Pst I
Hha I
Pst I
Hha I
Hinf I
Sma I
Tac I — Hha I
Pst I
Pst I — Pvu II
Pst I *

Pst I

"EcoR I".
Pst I
Sma I
Bal — Pvu II
I — Bgl II
Hinc II
Sma I
Sma I
Sst I — Taq I
Xho I — Pvu II
Bal I
Xba I
Acc I
Taq I
Xho I
Xba I
Hind III — Acc I
Xho I
Sst I
Hind III
Pst I
Bal I — Bgl II
Eco R I

5'
3'

NO SITES:

Bam H I
Kpn I
Sal I
Hpa I
Pvu I

■ intervening sequences
▨ coding sequence
☐ untranslated sequences

2/2

FIG. 2

pg PORK INSULIN

PERCENT DISPLACEMENT

CLONE 3-6 (50/1)

CLONE 3-1 (50/1)

CLONE 6-4 (200/1)

39 = 80pg

58 = 30pg

70.5 = 18pg